# FASCICULE DE BREVET EUROPEEN

(11) **EP 3 483 595 B1**
(45) Date de publication et mention de la délivrance du brevet: **18.03.2020**
(21) Numéro de dépôt: 18202426.5
(22) Date de dépôt: 24.10.2018
(51) Int. Cl.: G01N 23/044, A61B 6/02, G06T 7/55

(54) **DISPOSITIF ET PROCÉDÉ D'INSPECTION TRIDIMENSIONNELLE D'UN OBJET PAR RAYONS X**
VORRICHTUNG UND VERFAHREN ZUR 3D-INSPEKTION EINES OBJEKTS MIT RÖNTGENSTRAHLEN
DEVICE AND METHOD FOR THREE-DIMENSIONAL INSPECTION OF AN OBJECT USING X-RAYS

(30) Priorité: 09.11.2017 FR 1760539
(43) Date de publication de la demande: 15.05.2019
(73) Titulaire: Commissariat à l'Energie Atomique et aux Energies Alternatives, 75015 Paris (FR)
(72) Inventeur: PRAS, Philippe, 91460 MARCOUSSIS (FR); ROSSE, Bertrand, 91530 SAINT MAURICE MONTCOURONNE (FR); RAYBAUT, Pierre, 92160 ANTONY (FR)
(74) Mandataire: Santarelli

(56) Documents cités:
- WO-A1-2009/153789
- DE-A1- 4 235 183
- US-A- 6 028 910
- US-A1- 2003 043 962
- US-A1- 2015 221 080

## Description

L'invention porte sur le domaine de l'inspection tridimensionnelle d'objets par rayon X, en particulier par radiographie numérique. Elle s'applique notamment à des objets ne pouvant être déplacés et/ou présentant de grandes dimensions relativement aux dimensions de l'imageur pouvant être employé.

En particulier, l'invention porte sur un dispositif d'inspection tridimensionnelle d'un objet par rayons X, notamment un dispositif mobile, et sur un procédé d'utilisation d'un tel dispositif.

Dans le domaine de la radiographie mobile, les systèmes connus dans l'état de la technique peuvent être distingués et classés en prenant en compte deux critères principaux, à savoir la qualité des images qu'ils permettent d'obtenir et la facilité de leur mise en oeuvre. La qualité des images fait référence notamment à la résolution du système, sa sensibilité, et le rapport signal / bruit. La facilité de mise en oeuvre fait référence à des caractéristiques telles que la masse et le volume du dispositif, le temps nécessaire à sa mise en oeuvre tant pour son installation que lors des mesures, et sa robustesse à l'environnement d'utilisation.

En plus de ces critères, il est bien souvent souhaitable de pouvoir mesurer la profondeur, dans l'objet, de sous-éléments de l'objet radiographié. On parle alors d'inspection tridimensionnelle de l'objet.

Deux grandes familles de systèmes connus peuvent être envisagées pour réaliser de la radiographie mobile, à savoir les systèmes d'écrans radio luminescents à mémoire (souvent désignés par l'acronyme ERLM) d'une part, et les imageurs numériques d'autre part.

Pour la radiographie d'objets de grandes dimensions, les systèmes d'écrans radio luminescents à mémoire sont bien adaptés. En effet, les systèmes connus employant cette technologie sont suffisamment légers pour couvrir des surfaces importantes par juxtaposition de plusieurs écrans. Néanmoins ces systèmes offrent un rapport signal sur bruit et une sensibilité généralement inférieurs à ceux proposés par les meilleurs imageurs numériques de l'état de l'art. En outre, comme la lecture de ces écrans est différée, il faut changer manuellement de configuration d'exposition (générateur et/ou imageur) pour obtenir une information tridimensionnelle. Ceci nuit à la rapidité d'opération.

Les imageurs numériques de radiographie, également appelés imageurs X numériques (en référence aux rayons X employés), permettent l'obtention instantanée d'images. L'invention ici développée met en oeuvre cette technologie.

Les images obtenues par ces systèmes sont néanmoins de dimensions réduites comparativement à une juxtaposition d'images pouvant être obtenue dans un système ERLM. En outre, dans l'état de la technique, les systèmes connus sont incompatibles de la radiographie d'objets de grandes dimensions relativement à la taille de l'imageur, ou présentent des inconvénients en termes de facilité d'utilisation ou de qualité d'image.

Pour ce qui concerne la tomographie par rayon X, qui par nature concerne la reconstruction tridimensionnelle d'objets, plusieurs documents sont connus dans l'état de la technique.

On connait notamment le document EP0037151 qui divulgue un appareil de tomographie assisté d'un ordinateur. Le document EP 1356433 décrit un procédé de reconstruction d'image tomographique. Le document EP18338212 décrit un appareil et un procédé tomographique en cohérence optique et détection combinée.

On connaît également les documents US2015/221080 et US2003/043962 qui divulguent des systèmes de tomosynthèse.

De manière générale, dans les approches de reconstruction tridimensionnelle d'objets envisagées dans l'état de la technique, un compromis est nécessaire entre la masse et le volume du système d'une part, et le temps nécessaire à l'acquisition d'autre part. En effet, soit les moyens de détection et/ou d'irradiation sont augmentés en nombre dans le système, ce qui permet des mesures en un temps limité, mais est pénalisant en termes de masse et de volume du système employé, soit on emploie un nombre limité de moyens de détection et/ou d'irradiation que l'on déplace pour effectuer de multiples mesures autour de l'objet, mais cela est pénalisant en termes de durée d'acquisition et de complexité de mise en oeuvre du système.

Pour la reconstruction tridimensionnelle de petits objets, les systèmes employés peuvent être mobiles parce qu'ils mettent en oeuvre de petits générateurs de rayons X et de petits imageurs numériques. Cela est par exemple le cas dans les applications d'orthodontie pour lesquelles les diagnostics des patients doivent être rapides. Un seul détecteur numérique peut être employé dans ce cas, avec plusieurs sources de rayons X. Les documents suivants décrivent de telle applications :
US 7,440,540 82, selon lequel deux sources de rayons X espacées de la largeur des yeux irradient une dent d'intérêt que l'on visualise comme une image tridimensionnelle obtenue dans le spectre visible ; et
US 8,855,393 82, selon lequel trois sources de rayons X sont convergentes sur une dent d'intérêt qui est modélisée numériquement par un post-processeur.

Dans les applications de tomographie par rayons X de zones plus larges du corps humain, un seul détecteur est généralement employé, de dimension suffisante.

Le document US 6,236,708 associe ainsi un seul détecteur à un générateur de rayons X qui se déplace sur un arc de cercle centré sur la partie du corps à diagnostiquer. La multitude d'images réalisées permet de modéliser la partie radiographiée pour la visualiser. Les moyens mécaniques de déplacement nécessaires sont imposants et impliquent que ce soit le patient qui se rapproche du système, et non l'inverse.

Le document US 2013/0163719 divulgue un dispositif analogue, optimisé en ce qui concerne sa mobilité afin de pouvoir être déplacé dans les couloirs d'un hôpital. Pour cela, deux générateurs de rayons X espacés d'une largeur fixe sont associés à un seul imageur. La visualisation en trois dimensions est directe et ne nécessite pas un post-processeur pour l'obtenir.

Néanmoins, un tel système ne permet pas la radiographie d'objets de grandes dimensions, en ce que la taille des objets qu'il peut radiographier est limitée par la dimension de son imageur. En outre, le système n'est pas suffisamment robuste pour certaines applications, notamment celles liées à la sécurité.

Pour la radiographie d'objets de très grandes dimensions, notamment de véhicules, on connait par exemple par le document US 7,819,58082 un portique de radiographie, qui est fixe, et sous lequel l'objet à radiographier est déplacé.

Pour le contrôle des bagages, par exemple dans un aéroport, le document US 6,904,122 propose de n'utiliser qu'un seul imageur et qu'un seul générateur de rayons X dont on valorise la divergence. Pour cela, le cône d'irradiation du générateur de rayons X est collimaté pour créer deux pinceaux de rayons X issus de la même source. Le déplacement de l'objet généré par le convoyeur de bagages permet de multiplier les vues et obtenir l'information 3D recherchée. A cause des collimateurs et du convoyeur, ce système est nécessairement fixe.

Pour les applications de sécurité dans lesquelles il n'est pas possible de déplacer l'objet à contrôler, la problématique de l'obtention de la profondeur des objets visualisés en imagerie par rayons X en deux dimensions se pose également. Pour des objets ayant une surface projetée inférieure à la taille de l'imageur numérique, il suffit selon le document US 8,976,926 de déplacer le générateur de rayons X pour obtenir cette information. Dans le système décrit dans ce document, le générateur de rayons X se déplace à l'extrémité d'un bras mobile sur un arc de cercle dans un plan parallèle au plan de l'imageur. Les images obtenues sont superposées dans un plan appelé « plan de convergence » pour l'objet d'intérêt. La méthode ainsi proposée permet de minimiser le nombre de vues nécessaires à l'obtention de l'information tridimensionnelle recherchée, mais elle ne convient pas pour la radiographie des objets beaucoup plus grands que l'imageur, à moins de répéter de nombreuses fois le procédé visé dans ce document.

Un système d'imagerie par rayons X de grande dimension a été imaginé pour obtenir la radiographie complète de conteneurs imposants au moyen d'imageurs numériques de dimensions réduites. Un tel système est décrit par le document US 7,800,061, selon lequel le générateur et l'imageur sont suspendus par deux systèmes de câbles afin d'être toujours en regard l'un de l'autre autour du conteneur. La mécanique des câbles s'appuie sur le conteneur à contrôler qui doit être suffisamment solide pour porter l'imageur et le générateur de rayons X. Cette méthode est inadaptée pour les applications de sécurité qui exigent rapidité, adaptabilité et absence de contact physique avec l'objet à contrôler.

Il ressort que, dans l'état de la technique concernant l'inspection tridimensionnelle d'objets par rayon X, il n'y a pas de système connu permettant l'inspection d'objets de grandes dimensions par un dispositif mobile, tout en offrant une bonne qualité d'image et une grande facilité d'utilisation.

L'invention développée tend à résoudre tout ou partie des inconvénients précités.

Ainsi, l'invention porte sur un dispositif d'inspection tridimensionnelle d'un objet par rayons X, comportant un générateur de rayons X et un imageur numérique rectangulaire ou carré en regard dudit générateur de rayons X. La distance entre le générateur de rayons X et l'imageur définit le grandissement de 1, le générateur de rayons X étant positionné sur un axe orthogonal à un plan d'extension de l'imageur, passant par le centre de l'imageur. Le générateur de rayons X est mobile dans un plan parallèle audit plan d'extension de l'imageur, et l'imageur est mobile dans son plan d'extension.

Le dispositif comporte en outre un moyen de contrôle de la position et du déplacement dudit générateur de rayons X et dudit imageur configuré de sorte à déplacer le générateur de rayons X par déplacements unitaires selon une première direction ou une deuxième direction respectivement parallèles aux côtés de l'imageur, et pour maintenir à chaque déplacement unitaire la position relative de l'imageur par rapport audit générateur de rayons X, le moyen de contrôle étant configuré de sorte que chaque déplacement unitaire du générateur de rayons X et de l'imageur, dans la première ou dans la deuxième direction, corresponde à une fraction entière du côté correspondant de l'imageur (à savoir 1/N fois la dimension du coté correspondant à la première direction et 1/N fois la dimension du côté correspondant à la deuxième direction, N étant un entier naturel supérieur à 1), de sorte à obtenir par prises de vue successives après chaque déplacement unitaire un ensemble matriciel de sous-images se chevauchant dans le plan d'extension de l'imageur.

Dans un tel dispositif, chaque sous-image a un centre affecté de coordonnées dans un plan de grandissement égal à N, lesdites coordonnées étant déterminées selon la position du centre de l'imageur lors de la prise de ladite sous-image, le dispositif comportant en outre des moyens de traitement d'image adaptés à réaliser un agrandissement homothétique de chaque sous-image, laissant invariantes les coordonnées du centre de chaque sous-image, lesdits moyens étant en outre configurés pour déterminer un facteur d'étirement permettant la coïncidence des sous-images représentant tout ou partie d'un élément d'intérêt prédéfinie de l'image.

Le déplacement coordonné du générateur de rayons X et de l'imageur permet l'obtention au niveau de l'imageur d'images se recoupant en partie au moins dans une zone d'intérêt. La multiplicité des informations bidimensionnelles dans cette zone permet de déterminer sa profondeur dans l'objet radiographié. L'invention permet ainsi de bénéficier de la qualité d'image des imageurs numériques, par exemple en termes de sensibilité et de résolution, notamment dans des situations où il n'est pas possible ou pas souhaité de déplacer l'objet inspecté. Elle vise notamment l'inspection des objets de grandes dimensions. En adaptant les déplacements unitaires du générateur et de l'imageur, l'invention permet une adaptation aux situations dans lesquelles seul un faible recul vis-à-vis de l'objet inspecté est disponible. L'invention permet l'obtention d'une information tridimensionnelle de manière simple et rapide.

Le dispositif peut comporter des moyens de détermination d'une profondeur et des dimensions de l'élément d'intérêt en fonction du facteur d'étirement déterminé.

Le dispositif peut comporter un premier support portant l'imageur et un deuxième support portant le générateur de rayons X.

Par exemple, le premier support peut comporter des roulettes motorisées, configurées pour déplacer l'imageur dans la première direction, et un moyen de positionnement dudit imageur dans la deuxième direction comportant un élément télescopique ou un bras le long duquel l'imageur peut se translater, et le deuxième support peut comporter des roulettes motorisées, configurées pour déplacer le générateur de rayons X dans la première direction, et un moyen de positionnement dudit générateur de rayons X dans la deuxième direction comportant un élément télescopique ou un bras le long duquel le générateur de rayons X peut se translater.

Dans un autre mode de réalisation, le premier support comporte un premier portique et le deuxième support comporte un second portique, le premier portique comportant une première poutre s'étendant dans la première direction, et un élément portant l'imageur s'étendant dans la deuxième direction adapté à positionner ledit imageur dans ladite deuxième direction, ledit élément portant l'imageur étant lié à la première poutre de sorte à pouvoir être translaté selon la première direction, ledit élément portant l'imageur étant un élément télescopique ou une seconde poutre le long de laquelle l'imageur peut se translater. Le deuxième portique peut alors avoir une configuration identique à celle du premier portique et portant le générateur de rayons X en lieu et place de l'imageur du premier portique.

Dans un autre mode de réalisation, le premier support comporte un premier portique et le deuxième support comporte un second portique, le premier portique comportant un premier élément télescopique s'étendant dans la première direction, et un second élément télescopique portant l'imageur s'étendant dans la deuxième direction adapté à positionner ledit imageur dans ladite deuxième direction, ledit deuxième élément télescopique étant lié au premier élément télescopique de sorte à être déplacé dans la première direction par extension ou rétractation dudit premier élément télescopique, et le deuxième portique ayant une configuration identique à celle du premier portique et portant le générateur de rayons X en lieu et place de l'imageur du premier portique.

Le premier et le deuxième support peuvent comporter chacun un pied anti-basculement comportant une troisième poutre, liée rigidement sensiblement orthogonalement à la première poutre.

Le dispositif peut comporter un dispositif d'alignement à laser permettant l'alignement relatif des premier et deuxième supports.

Dans un autre mode de réalisation, le dispositif comporte une structure liant rigidement le générateur de rayons X et l'imageur, ladite structure entre le générateur de rayons X et l'imageur étant portée par un support comportant un système de déplacement de ladite structure selon la première direction et selon la deuxième direction. Le système de déplacement de la structure peut comporter des roulettes motorisées configurées pour déplacer la structure dans la première direction, et un mécanisme comportant un élément télescopique lié à la structure ou un bras orienté selon ladite deuxième direction le long duquel la structure peut se translater.

L'invention porte également sur un procédé d'inspection tridimensionnelle d'un objet par rayons X, comportant les étapes de :
- fourniture d'un dispositif tel que précédemment décrit ;
- mise en place du dispositif vis-à-vis de l'objet, le générateur de rayons X et l'imageur étant espacés l'un de l'autre d'une distance correspondant au grandissement de 1 ;
- obtention d'un ensemble matriciel de sous-images ;
- détermination d'un élément d'intérêt de l'objet représenté dans ledit ensemble de sous- images ;
- détermination, par agrandissement homothétique et superposition partielle des sous-images représentant tout ou partie de l'élément d'intérêt, d'un facteur d'étirement permettant la coïncidence des sous-images au niveau de l'élément d'intérêt ; et
- détermination d'une profondeur et des dimensions de l'élément d'intérêt en fonction du facteur d'étirement déterminé.

Ce procédé peut en outre comporter, entre la fourniture du dispositif et sa mise en place,
- une étape de détermination du champ de vue nécessaire pour radiographier l'objet,
- une étape de détermination du recul disponible vis-à-vis d'une face avant de l'objet pour la mise en place du générateur de rayon X ; et
- une étape de détermination de N en fonction du recul disponible et des dimensions d'une zone d'intérêt contenant l'élément d'intérêt.

Dans un tel procédé, N peut être déterminé de sorte que :
- si la zone d'intérêt est de dimension supérieure dans la première direction à la fraction 1/N du côté de l'imageur correspondant à la première direction et est de dimension supérieure dans la deuxième direction à la fraction 1/N du côté de l'imageur correspondant à la deuxième direction, N est le plus petit entier, supérieur à 1, pour lequel le recul est supérieur à 1/N fois la distance entre le générateur de rayon X et l'imageur,
- si la zone d'intérêt est de dimension inférieure dans la première direction à la fraction 1/N du côté de l'imageur correspondant à la première direction ou est de dimension inférieure dans la deuxième direction à la fraction 1/N du côté de l'imageur correspondant à la deuxième direction, N est le plus petit entier pour lequel le recul est supérieur à 2/N fois la distance entre le générateur de rayon X et l'imageur.

Le procédé peut également comprendre une étape de détermination du nombre de vues n nécessaires avec l'imageur pour couvrir le champ de vue, sans superposition d'images, dans la première direction et du nombre de vues m nécessaires avec l'imageur pour couvrir le champ de vue, sans superposition d'image, dans la deuxième direction, et pour N=2 ou N=3, l'étape d'obtention de l'ensemble matriciel de sous-images comprend l'obtention de N.n-3 à N.n+1 sous-images dans la première direction et de N.m-3 à N.m+1 sous-images dans la deuxième direction.

D'autres particularités et avantages de l'invention apparaîtront encore dans la description ci-après :
Aux dessins annexés, donnés à titre d'exemples non limitatifs :
- la figure 1 illustre sur un diagramme le principe mis en oeuvre dans l'invention par un premier exemple d'application ;
- la figure 2 illustre sur un diagramme analogue à celui de la figure 1 un second exemple d'application ;
- la figure 3 illustre sur un diagramme analogue à celui des figures 1 et 2 un troisième exemple d'application ;
- la figure 4 illustre de manière schématique un aspect de l'invention ;
- la figure 5 illustre selon une vue schématique en trois dimensions un dispositif conforme à un mode de réalisation de l'invention ;
- la figure 6 illustre selon une vue schématique en trois dimensions un dispositif conforme à un mode de réalisation de l'invention ;
- la figure 7 illustre selon une vue schématique en trois dimensions un dispositif conforme à un mode de réalisation de l'invention ;
- la figure 8 illustre selon une vue schématique en trois dimensions un dispositif conforme à un mode de réalisation de l'invention ;
- les figures 9a à 9c illustrent le traitement d'image réalisé dans un mode de réalisation de l'invention ;
- la figure 10 illustre sous forme d'un logigramme les étapes d'un procédé conforme à un mode de réalisation particulier de l'invention.

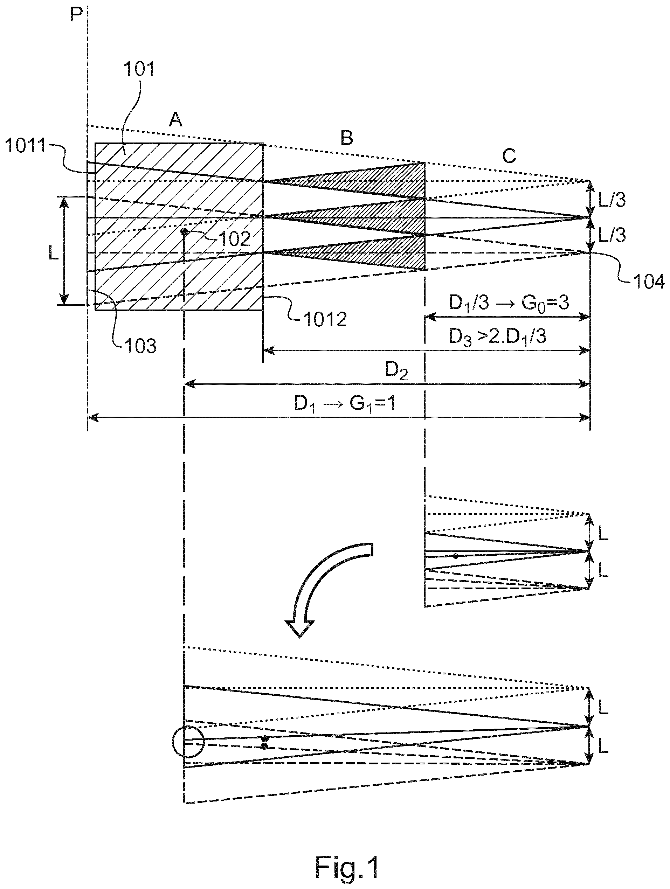

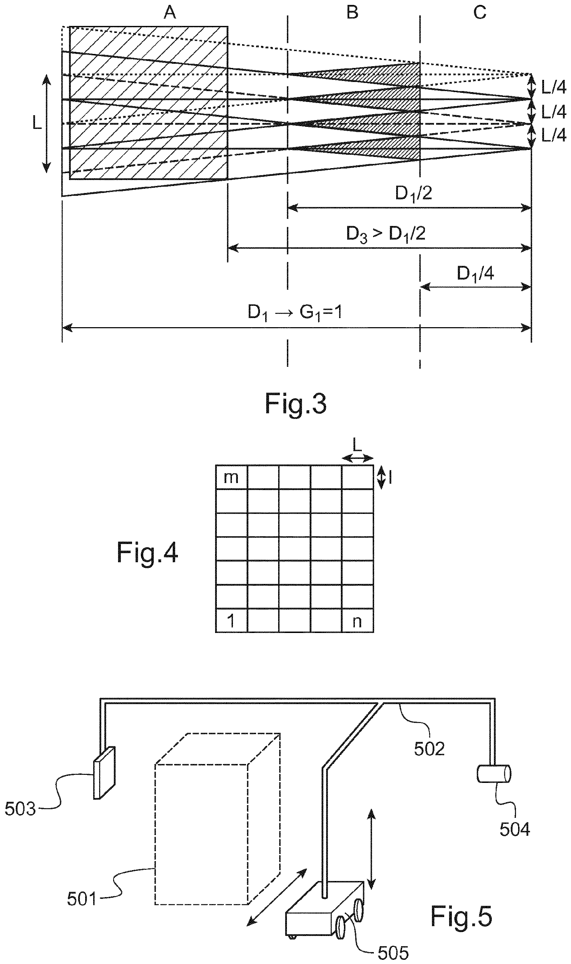

La figure 1 illustre sur un diagramme le principe mis en oeuvre dans l'invention par un premier exemple d'application. L'invention met en oeuvre un dispositif dont des exemples détaillés de modes de réalisation sont décrits plus en détail en référence aux figures 5 à 8.

A la figure 1, l'objet 101 à inspecter, c'est-à-dire l'objet que l'on souhaite contrôler ou diagnostiquer est vu de dessus. L'objet 101 est ici représenté sous la forme d'un rectangle : cet objet peut par exemple présenter une forme sensiblement parallélépipédique droite.

A l'intérieur de l'objet 101, un élément d'intérêt doit être radiographié avec précision. Il s'agit dans l'exemple ici représenté d'un élément ponctuel, constituant un point d'intérêt 102. La profondeur du point d'intérêt 102 dans l'objet 101 doit être déterminée dans le cadre de l'inspection.

Selon le principe développé dans l'invention, on place un imageur X numérique 103 à proximité de l'objet, par exemple à proximité d'une face arrière 1011. Un générateur de rayons X 104, qui peut également être appelé «source» est positionné en regard d'une face avant 1012 de l'objet 101, de manière adéquate par rapport à l'imageur. En particulier, le générateur de rayon X 104 est positionné sur l'axe passant par le centre de l'imageur et orthogonal à celui-ci (c'est-à-dire orthogonal au plan d'extension P de l'imageur 103), de l'autre côté de l'objet 101 que l'imageur 103. La distance entre l'imageur 103 et le générateur de rayons X 104 est référencée D1.

La distance D1 entre le générateur de rayons X 104 et l'imageur 103 est telle qu'il n'y a pas de grandissement dans le plan d'extension P de l'imageur 103, ou, autrement dit, que le grandissement dans ce plan d'extension P référencé G₁ est égal à 1.

En partant de l'hypothèse que l'imageur 103 présente une surface sensible rectangulaire, on forme ainsi dans l'espace une pyramide (matérialisée sur la figure 1) dont le sommet est le générateur de rayons X 104 (qui désigne par abus de langage, ici et dans le reste de la présente description, le point d'émission ou « sortie » dudit générateur en tant que dispositif), et la base est l'imageur 103 (qui désigne par abus de langage, ici et dans le reste de la présente description, la surface sensible de l'imageur en tant que dispositif). L'imageur présente une dimension L dans une première direction (par exemple la largeur de l'imageur 103) et une dimension I dans une deuxième direction orthogonale à la première direction (par exemple la hauteur de l'imageur 103)

Le générateur de rayons X 104 est à une distance D3 du point extrême avant de l'objet 101, c'est-à-dire de sa face avant 1012 dans l'exemple ici représenté.

La distance, inconnue de manière précise avant application de l'invention, entre le générateur de rayons X 104 et le point d'intérêt 102 est référencé D2. Plus précisément, D2 correspond à la distance la plus courte entre le plan parallèle au plan d'extension P de l'imageur 103 passant par le générateur de rayons X 104 et le point d'intérêt 102.

Le grandissement avec lequel le point d'intérêt est vu sur l'imageur 103 est G₂=D₁/D₂.

Des prises de vue successives de l'objet sont réalisées à l'aide de l'imageur. Entre chaque prise de vue, l'imageur 103 est déplacé dans son plan d'extension, selon une première direction (par exemple horizontalement) ou selon une seconde direction (par exemple verticalement). Le générateur de rayons X 104 est déplacé en correspondance, simultanément ou non, de sorte que sa position relative par rapport à l'imageur 103 est inchangée à chaque prise de vue.

Chaque déplacement de l'imageur 103, dans la première ou la deuxième direction, correspond à une fraction entière prédéfinie de la dimension dudit imageur 103 dans cette direction. Par exemple, pour un imageur rectangulaire de largeur L, chaque déplacement unitaire de l'imageur 103 dans la direction parallèle aux cotés de l'imageur de largeur L correspond à une distance de 1/N fois la largeur L (avec N entier naturel supérieur à 1). Si l'imageur rectangulaire a une hauteur I (dimension perpendiculaire à sa largeur), chaque déplacement unitaire de l'imageur dans la direction parallèle aux cotés de l'imageur de hauteur I correspond à une distance de 1/N fois la hauteur I).

Conséquemment, le générateur est déplacé en correspondance.

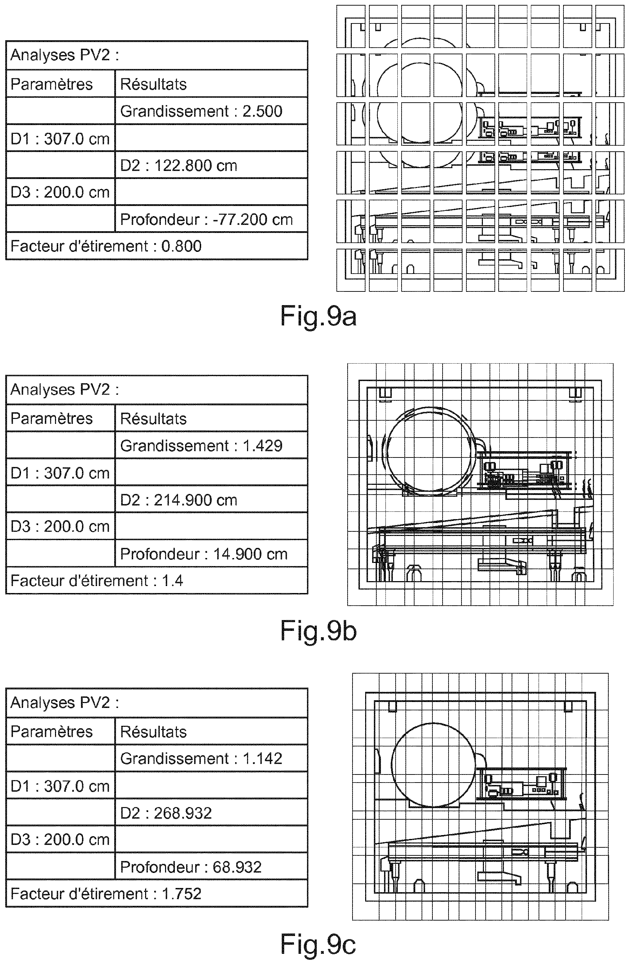

Des déplacements unitaires sont ainsi réalisés dans une première direction et une deuxième direction orthogonale à la première direction, et à chaque fois une prise de vue est réalisée. Chaque vue est indexée selon ses coordonnées, par exemple les coordonnées de son centre dans le plan d'extension P de l'imageur 103. On obtient ainsi un ensemble matriciel de sous-images, dont un exemple est décrit ci-après en référence à la figure 9a.

Afin d'inspecter un objet 101 de grande dimensions, une multitude de vues est nécessaire. En fonction des dimensions de l'objet, on peut déterminer les dimensions du champ de vue nécessaire à son inspection, c'est-à-dire les dimensions dans le plan de l'imageur qui doivent être couvertes par l'ensemble des sous-images.

Par exemple le champ de vue nécessaire peut avoir une dimension de n fois L (L étant la dimension de l'imageur 103 dans la première direction) et une dimension de m fois I (I étant la dimension de l'imageur 103 dans la deuxième direction). Cet exemple est illustré à la figure 4. Si N était égal à 1 (ce qui est exclu de l'invention, et correspondrait à une simple reconstitution d'images sur la base de sous-images qui ne se superposent pas) il faudrait n fois m sous-images (et donc autant de prises de vue) pour radiographier au moins une fois l'arrière de l'objet. Pour tout N entier naturel définissant la fraction de la dimension de l'imageur dans la première direction correspondant à un déplacement unitaire dans ladite première direction, il faut typiquement N.n positions pour analyser le contenu de l'objet dans la première direction. De manière analogue, dans la deuxième direction il faut typiquement N.m positions pour analyser le contenu de l'objet dans la deuxième direction. Le nombre de prises de vues nécessaires doit néanmoins être adapté au champ de vue à couvrir, selon les dimensions de l'objet et selon la zone dans laquelle on souhaite réaliser les prises de vue, entre une zone dite « A » et une zone dite « B » définies ci-après. Le nombre de prise de vue est notamment adapté pour ne pas perdre d'information sur la face avant de l'objet. Ainsi, un sous ensemble de N.n par N.m sous-images est souvent nécessaire pour couvrir l'ensemble du champ de vue, mais le nombre de sous-images peut en pratique varier de N.n-3 à N.n+1 dans la première direction et de N.m-3 à N.m+1 dans la seconde direction, pour N=2 ou N=3.

En généralisant, pour tout entier 1 ≤ a ≤ N , si l'objet à une hauteur m-a/N < h/L ≤ m-(a-1)/N, il faut N.m+2-a positions pour la zone dite « A » et N.m+1-a positions pour la zone dite « B ». Certaines contraintes mécaniques d'un sol ou d'un mur peuvent amener à retirer 1 position par rapport à ces valeurs.

A la figure 1, N est égal à trois. Dans la première direction, chaque déplacement de l'imageur et corolairement du générateur dans la première direction correspond ainsi au tiers de la largeur L de l'imageur 101, c'est-à-dire à L/3.

A la figure 1, on a représenté l'imageur 103 et le générateur de rayons X 104 respectivement dans trois positions, chacune séparée dans la première direction d'une distance de L/3. Les trois positions sont représentées respectivement en trait plein, en traits pointillés longs, et en traits pointillés courts. Les pyramides formées entre le générateur de rayons X 104 et l'imageur 103 sont vues de dessus, et ont ainsi une forme triangulaire ayant pour axe de symétrie la droite orthogonale au plan d'extension P de l'imageur passant par le générateur de rayon X 104.

Dans la direction parallèle au plan d'extension P et orthogonale à la première direction (correspondant à la hauteur de l'imageur dans l'exemple représenté), l'imageur 103 et le générateur de rayons X 104 ont un mouvement unitaire analogue, du tiers de la hauteur I de l'imageur 104.

On définit ainsi trois zones :
- une première zone A, pour laquelle chaque point de l'objet est radiographié au moins deux fois à deux dimensions (au moins quatre fois à trois dimensions moyennant les fractions de déplacement orthogonal) ;
- une deuxième zone B, dans laquelle chaque point est radiographié au moins une fois, et exactement une fois dans les zones hachurées de la figure 1 ; et
- une troisième zone C, où tous les points ne sont pas radiographiés, et les points radiographiés le sont une seule fois.

Dans l'exemple représenté à la figure 1, avec N égal à trois, la première zone A est celle pour laquelle la distance D au générateur de rayon X est telle que D> 2.D₁/3.

La deuxième zone B est celle pour laquelle la distance D au générateur de rayon X est telle que D₁/3 < D ≤ 2.D₁/3.

La troisième zone C est celle pour laquelle la distance D au générateur de rayon X est telle que D ≤ D₁/3.

En toute rigueur D est une distance la plus courte au plan parallèle au plan d'extension P, passant par le générateur de rayons X 104.

Dans l'exemple ici représenté, la face avant 1012 de l'objet 101 est positionnée à l'entrée de la première zone A, c'est-à-dire que D₃=2D₁/3.

L'ensemble matriciel de sous-images obtenues tel que précédemment expliqué peut ensuite faire l'objet d'un traitement d'image permettant la détermination de la distance du point d'intérêt 102 de la profondeur du point d'intérêt 102 dans l'objet 101.

La profondeur du point d'intérêt 102 dans l'objet 101 correspond à D₂-D₃.

Les sous-images ou images partielles obtenues sont considérées dans le plan de grandissement G₀=N. Ces traitements peuvent être automatisés ou réalisés visuellement par un opérateur. Pour ce qui concerne le traitement visuel appliqué, dont les étapes correspondantes peuvent être automatisées, on peut se référer aux figures 9a à 9c qui sont décrites plus en détail ci-après.

Les sous-images sont ainsi visualisées dans le plan de grandissement G₀. Pour cela, les images sont visualisées en transparence ou « calque », c'est-à-dire que les sous-images sont visualisées les unes superposées aux autres, une sous-image de niveau inférieur restant visible par transparence de la ou des sous-images de niveau supérieur. La position du centre des sous-images est maintenue dans le plan de grandissement G₀. Ainsi, comme l'illustre la partie centrale de la figure 1, les sous-images sont juxtaposées. Les sommets des pyramides, correspondant aux centres des sous-images ; sont espacés d'une distance L dans la première direction et d'une distance I dans la deuxième direction (seule la première direction est visible sur la figure 1).

Chaque sous-image est étirée de façon homothétique à partir de son centre dont la position reste invariante. Pour un facteur d'étirement F les projections du point d'intérêt sur plusieurs sous-images coïncident. Une image dans le plan du point d'intérêt 102 est alors obtenue, telle qu'illustrée en partie basse de la figure 1.

Le facteur d'étirement F peut être identifié de façon visuelle et interactive en faisant varier continument le facteur d'étirement au moyen d'un logiciel dédié jusqu'à ce que les deux sous-images coïncident au niveau du point d'intérêt. Une automatisation de cette fonction est possible.

Le grandissement G₂ dans le plan du point d'intérêt, est lié au facteur d'étirement F par la relation F = N/G₂.

La profondeur P du point d'intérêt est alors déterminée par la formule P=F.D₁/N-D₃.

Le principe décrit ci-dessus peut être appliqué pour tout point d'intérêt observé sur au moins deux sous-images. Ce principe est donc valable pour tout point de l'objet 101 qui appartient à la première zone A. Il n'est applicable que pour certains points de la deuxième zone B, à savoir les points présents dans une zone non hachurée de la figure 1.

Il en résulte que pour l'analyse d'éléments d'intérêts ponctuels (par exemple la visualisation de défauts ponctuels) le choix de N=3 est bien adapté, si le recul est suffisant pour que le point d'intérêt soit de façon certaine dans la première zone A. Dans le cas contraire le choix d'une fraction entière plus petite (N>3) est possible comme expliqué ci-après plus en détail, en référence à la figure 3.

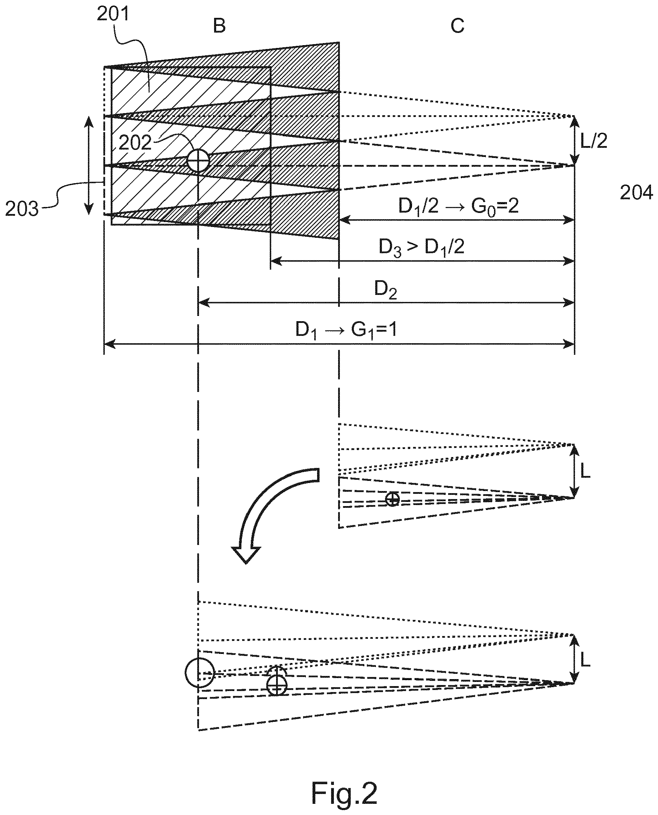

Dans le cas particulier où l'élément d'intérêt est de plus grande dimension, il est possible d'utiliser N=2, dans les conditions et selon les modalités expliquées en référence à la figure 2.

La figure 2 représente ainsi une optimisation du principe décrit en référence à la figure 1 quand l'élément d'intérêt 202 dans l'objet 201 (ou les éléments d'intérêts 202) est suffisamment grand. En particulier la dimension de l'objet 201 soit dans la première direction, soit dans la deuxième direction, doit être au moins égale à la fraction Nième de la dimension de l'imageur 203 dans cette direction. En d'autre termes, il faut que l'objet 201 soit plus grand que L/N dans la première direction, ou plus grand que I/N dans la deuxième direction.

Si cette condition est respectée, si l'élément d'intérêt se trouve dans la seconde zone B, il est certain que tout point de cet élément d'intérêt sera radiographié au moins une fois, et que certains points de l'élément d'intérêt seront radiographiés au moins deux fois, ce qui permet d'obtenir une information de profondeur pour ces points.

On notera que la deuxième zone B est utilisable dans les conditions précitées, que N soit égal à 2 comme illustré à la figure 2 ou que N soit égal à 3 comme illustré à la figure 1. On privilégiera un mode de réalisation dans lequel N=2 si le recul vis-à-vis de l'objet 101,201 est suffisant (en effet la deuxième zone B commence pour D>D₁/2 avec N=2) tandis qu'on peut utiliser N=3 si le recul est trop faible (la deuxième zone D commence pour D>D₁/3 avec N=3).

Si un recul encore plus faible est disponible, une valeur de N supérieure, par exemple N=4 comme illustré à la figure 3, peut être employé. La deuxième zone B commence de manière générale pour D>D1/N.

De même, l'augmentation de la valeur de N peut permettre de s'assurer qu'un élément d'intérêt (typiquement ponctuel) se situe dans la première zone A (qui commence pour D>2D₁/N).

Il est cependant souhaitable de minimiser autant que possible la valeur de N. En effet, le nombre de sous-images nécessaires pour couvrir l'ensemble du champ de vue souhaité est typiquement de N.n par N.m. Ainsi, le nombre de prise de vue augmente comme le carré de la valeur de N. Cela augmente d'autant la capacité de calcul nécessaire au traitement simultané des sous-images. En outre, il est de manière générale important de limiter autant que possible l'exposition de l'objet inspecté aux rayons X.

Le choix de N, le plus petit possible, est donc crucial pour minimiser la durée de mesure et la dose déposée sur l'objet.

Les figures 5 à 8 représentent des exemples de modes de réalisation d'un dispositif conforme à l'invention, permettant la mise en oeuvre des principes décrits ci-dessus en référence aux figures 1 à 4. Dans le cadre d'un dispositif mobile, dans tous les exemples présentés ci-après il est préférable de prévoir une alimentation par batteries, permettant un emploi nomade aisé du dispositif.

Les parties mobiles ainsi que la prise de vue peuvent être de préférence pilotées à distance, par des moyens filaires ou sans fil.

La figure 5 présente un premier exemple de mode de réalisation. Un objet 501 à inspecter est disposé sur un sol sensiblement horizontal.
Dans ce mode de réalisation le générateur de rayons X 504 et l'imageur 503 sont liés l'un à l'autre rigidement par une structure 502 qui les supportent.
La structure 502 est du type « en C », le générateur de rayons X 504 et l'imageurs 503 étant respectivement portés par une branche du « C » formé par la structure.
La structure 502 est portée par un système de déplacement selon la première direction et selon la deuxième direction. Le système de déplacement comporte un robot 505. Le robot 505 comporte des roulettes motorisées configurées pour déplacer la structure dans la première direction, et un mécanisme comportant des moyens de déplacer verticalement la structure 502. Par exemple, le robot 505 peut comporter un élément télescopique lié à la structure 502, ou un bras orienté selon la deuxième direction (ici la verticale) le long duquel la structure peut être translatée. Si un élément télescopique est employé, son déploiement ou sa rétraction permet le mouvement de l'élément qu'il porte selon son axe d'extension. Si un bras est employé, il peut porter une crémaillère ou un dispositif équivalent le long duquel l'élément porté peut se translater.

De manière générale, les éléments télescopiques employés dans ce mode de réalisation ainsi que dans les autres modes de réalisation de l'invention peuvent être des vérins linéaires (chaque élément télescopique étant formé d'un vérin ou comportant plusieurs vérins jumelés longitudinalement), ou des bras télescopiques comportant plusieurs segments mobiles en translation relative, et pouvant être disposés l'un dans l'autre ou l'un le long de l'autre. Plusieurs éléments télescopiques peuvent être aboutés pour offrir un plus grand débattement.

Les déplacements horizontaux et verticaux peuvent ainsi être indexés de façon précise par un robot 505 par exemple.

Le dispositif est configuré pour garantir que les déplacements s'effectuent bien dans un plan parallèle à l'imageur et que le générateur X pointe effectivement le centre de l'imageur sur un axe orthogonal à sa surface sensible.

Etant donnés les poids et volume des imageurs et générateurs de rayons X connus dans l'état de la technique, la configuration présentée à la figure 5 requiert de lester le robot de façon importante à cause du montage en porte à faux de la structure 502. En outre, une grande rigidité du dispositif est requise pour permettre la prise de sous-images nettes et une précision de positionnement suffisante pour permettre une bonne superposition des sous-images.

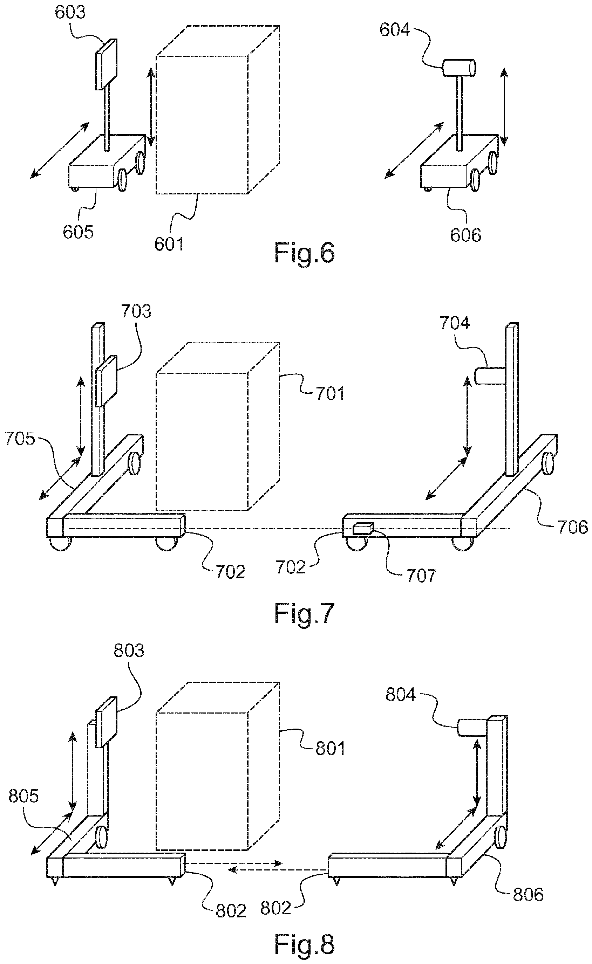

La figure 6 représente un autre mode de réalisation d'un dispositif conforme à l'invention. Dans ce mode de réalisation, un premier robot 605 forme un premier support pour l'imageur 603, et un deuxième robot 606 forme un deuxième support pour le générateur de rayons X 604. Le premier robot 605 et le deuxième robot 606 sont positionnés de part et d'autre de l'objet 601 à inspecter.

Les robots 602 et 603 sont aptes à se déplacer de façon analogue, ce qui déplace respectivement l'imageur numérique 603 et le générateur de rayons X 604.

Dans cette configuration, les déplacements ne sont pas nécessairement synchrones, mais le positionnement relatif adapté entre l'imageur 603 et le générateur de rayons X 604 doit être assuré à chaque prise de vue.

Les robots assurent, par leurs roulettes motorisées le déplacement horizontal de l'imageur 603 et du générateur de rayons X 604.

Pour le mouvement vertical, chaque robot est doté d'un élément télescopique ou d'un bras orienté selon ladite deuxième direction le long duquel l'imageur et le générateur X peuvent se translater.

La figure 7 représente un autre mode de réalisation d'un dispositif conforme à l'invention. Dans ce mode de réalisation, un premier support 705 porte l'imageur 703, et un deuxième support 706 porte le générateur de rayons X 704.

Les supports sont de type portique, et permettent un déplacement selon la première direction (par exemple horizontale, dans le plan d'extension de l'imageur) et dans la deuxième direction (par exemple verticale) de l'élément qu'ils portent.

Chaque support comporte pour cela une première poutre s'étendant dans la première direction, et une deuxième poutre portant soit l'imageur 703 soit le générateur de rayons X 704 s'étendant dans la deuxième direction. La deuxième poutre est liée à la première poutre de sorte à pouvoir être translatée selon la première direction. L'imageur 703 ou le générateur de rayons X 704 peut être translaté le long de la deuxième poutre, dans la deuxième direction.

Le premier support 705 et le deuxième support 706 sont appariés, et les mouvements de l'imageur 703 et du générateur de rayons X 704 sont synchronisés.

Les deuxièmes poutres verticales peuvent être remplacées par un élément télescopique vertical.

Les portiques comportent un pied anti-bascule 706, sensiblement orthogonal à la première direction et à la deuxième direction. Le premier portique et le deuxième portique peuvent être alignés l'un par rapport à l'autre, par exemple par alignement relatif de leurs pieds anti-bascule. Cet alignement peut être réalisé à l'œil, ou à l'aide d'un dispositif à laser 707.

Par exemple des lasers peuvent être placés aux extrémités des pieds anti-bascule 706 et être mécaniquement alignés avec eux. L'impact du laser sur une cible identifiée sur le pied du support en regard permet l'alignement relatif des premier et deuxième supports. Un seul laser 707 peut être employé, un miroir positionné sur le support opposé permettant d'effectuer un alignement par auto-collimation.

Dans l'exemple de mode de réalisation de la figure 7, les supports sont mobiles sur des roulettes multidirectionnelles afin d'amener le système auprès de l'objet et de faciliter les opérations d'alignement. Cette conception est peu chère par rapport à des robots multifonctions. En outre, l'espace requis derrière l'objet 701 (c'est-à-dire du côté de l'imageur) pour intercaler le premier support 705 entre l'objet et un obstacle tel qu'un mur est moins conséquent que dans la configuration représentée à la figure 6. En outre, la configuration présentée à la figure 7 permet un guidage linéaire parfaitement rectiligne le long des première et deuxième poutres.

La figure 8 représente un autre mode de réalisation d'un dispositif conforme à l'invention. Ce mode de réalisation est assez similaire dans sa configuration générale à celui représenté à la figure 7. Il comporte un premier support 805 qui porte l'imageur 803, et un deuxième support 806 qui porte le générateur de rayons X 804.

Les supports sont de type portiques, et permettent un déplacement selon la première direction (par exemple horizontale) et dans la deuxième direction (par exemple verticale) de l'élément qu'ils portent.

Dans ce mode de réalisation, les pieds anti-bascule 802 sont posés sur des supports articulés réglables en hauteur en lieu et place des roulettes du mode de réalisation de la figure 7.

Chaque support comporte un premier élément télescopique s'étendant dans la première direction sur lequel est fixé un deuxième élément télescopique s'étendant dans la deuxième direction. Dans l'exemple ici représenté, le deuxième élément télescopique est fixé en bout du premier élément télescopique. Une roulette de support permet le guidage linéaire horizontal lors du déploiement ou de la rétractation du premier élément télescopique. Un rail, non représenté, peut être mis en place pour guider la roulette.

Le dispositif de la figure 8 présente un encombrement très réduit pour son transport, tout en permettant des courses de déplacement de l'imageur et du générateur de rayons X importantes lors de son usage.

L'alignement des premier et deuxième supports peut être réalisé tel que décrit en référence à la figure 7.

Les dispositifs selon les différents modes de réalisation décrits, notamment ceux des figures 7 et 8, peuvent être dimensionnés de sorte à pouvoir être transportés sur une palette standard de 80 cm par 120 cm, ou dans le coffre d'un véhicule automobile de taille moyenne.

L'inspection d'un objet par un dispositif tel que précédemment décrit nécessite un dispositif de traitement d'images permettant la réalisation d'opérations en temps réel sur l'ensemble de sous-images obtenu.

Le logiciel d'analyse des images employé dispose de fonctionnalités telles que : la lecture et enregistrement d'une image, le réglage de contraste, la possibilité de zoomer pour visualiser des détails les plus fins, une fonction de superposition de formes (segments, cercles, ellipses, rectangles, etc.) de dimensions connues en nombre de pixels afin de mesurer des zones d'images.

Une fonction spécifique de traitement d'image permet de manipuler de façon interactive (déplacement, étirement et rotation à partir du centre, etc.) simultanément typiquement N.n par N.m images partielles. Chaque image peut avoir une taille de plusieurs Méga-octets. Un tel logiciel, peut être mis en oeuvre par un ordinateur, par exemple un ordinateur portable. Le logiciel et le système informatique employés doivent de préférence être capables de modifier de multiples images volumineuses simultanément avec un temps de calcul de l'ordre de quelques millisecondes afin que les modifications graphiques soient perçues sans délai par l'utilisateur.

Une interface graphique spécifique permet d'ouvrir l'ensemble des sous-images acquises et de les juxtaposer dans le plan de grandissement Go=N sur une matrice typique de N.n colonnes et N.m lignes (le nombre de lignes et de colonnes étant adapté au nombre de prises de vue effectives) que l'on appelle « mosaïque de sous-images ».

A la figure 9a on a représenté une mosaïque de sous-images issue de la radiographie d'un objet cubique d'un mètre cube contenant une boule de 36 cm de diamètre (qui correspond à l'élément d'intérêt) et des dispositifs électroniques. Dans cet exemple N=2.

A la figure 9a les sous-images sont représentées pour un facteur d'étirement F égal à 0,8. Les sous-images seraient juxtaposées les unes aux autres pour un facteur d'étirement F égal à 1, et un grandissement de 2.

Afin d'être capable de transformer des dimensions en pixels en dimensions en cm par exemple, le logiciel dispose d'une interface avec l'opérateur pour renseigner les valeurs de D1 et D3 mesurées physiquement pour la configuration de l'étude. Dans l'exemple ici représenté, l'interface graphique (à gauche) permet d'entrer les valeurs de D1 (ici 307 cm) et de D3 (ici 200 cm) qui correspondent à la configuration du dispositif lors de l'acquisition de l'ensemble de sous-images.

Le logiciel peut être configuré de sorte à offrir à l'utilisateur une bonne ergonomie et une grande facilité d'utilisation. Par exemple, la manipulation d'un curseur peut permettre de faire varier continument la taille de chaque sous-image d'un même facteur d'étirement F. Quand la superposition des sous-images est correcte au niveau d'un élément d'intérêt, le logiciel affiche le grandissement G associé au facteur d'étirement F courant et la profondeur P de cet élément d'intérêt dans l'objet inspecté.

A la figure 9b, le facteur d'étirement des 35 images a été porté à une valeur de 1,4 de sorte que le grandissement courant est égal à 1,429 et que la profondeur correspondante dans l'objet est de 14,9 cm.

La « mise au point » (c'est-à-dire la superposition correcte des sous-images) n'est pas encore effectuée dans le plan diamétral vertical de la boule.

A la figure 9c, le plan de convergence de la boule est atteint pour un facteur d'étirement F=1,752, c'est-à-dire pour un grandissement de 1,142 et une profondeur de près de 69 cm.

La superposition d'une forme dans le plan de convergence permet de mesurer la taille réelle de l'élément d'intérêt parce que son grandissement est connu. Dans l'exemple ici représenté, la superposition d'un cercle au disque représentant la boule permet de mesurer le diamètre de la boule à 36,1cm en se fondant sur le nombre de pixels par centimètre qui est connu pour le plan de grandissement G=2.

Tous les éléments de l'objet dont la mise au point est correcte sur l'image représentée à la figure 9c sont dans le plan diamétral vertical de la boule.

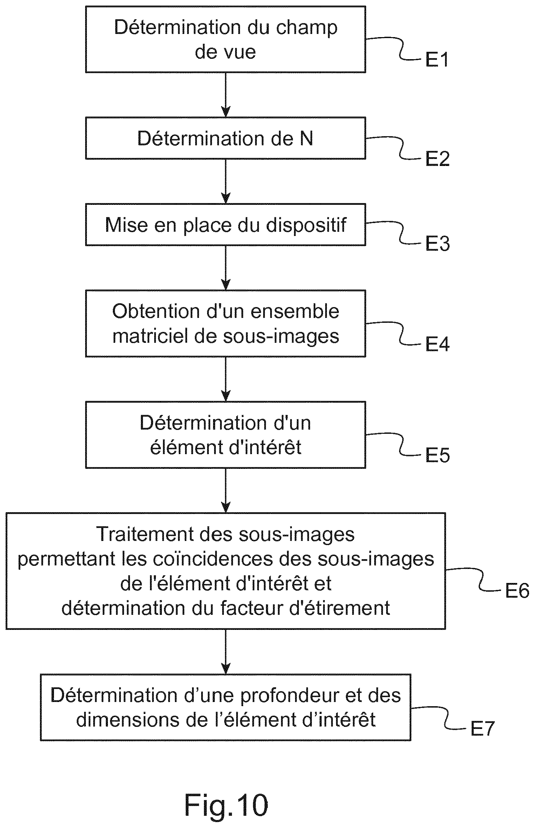

L'invention porte également sur un procédé d'inspection tridimensionnelle d'un objet par rayons X, dont un exemple particulier est illustré à la figure 10 qui représente la succession des étapes pouvant être mise en oeuvre.

Le procédé emploi un dispositif conforme à un mode de réalisation de l'invention.

Le procédé comporte une étape de détermination du champ de vue nécessaire pour radiographier un objet à inspecter (E1).

L'entier N est ensuite déterminé, en prenant en considération le recul disponible et des dimensions d'une zone d'intérêt contenant l'élément d'intérêt (E2). Cette étape peut être réalisée selon les modalités décrites ci-avant en référence aux figures 2 et 3. Il en découle que :
- si la zone d'intérêt est de dimension supérieure dans la première direction à la fraction 1/N du côté de l'imageur correspondant à la première direction et est de dimension supérieure dans la deuxième direction à la fraction 1/N du côté de l'imageur correspondant à la deuxième direction, N est le plus petit entier, supérieur à 1, pour lequel le recul est supérieur à 1/N fois la distance entre le générateur de rayon X et l'imageur,
- si la zone d'intérêt est de dimension inférieure dans la première direction à la fraction 1/N du côté de l'imageur correspondant à la première direction ou est de dimension inférieure dans la deuxième direction à la fraction 1/N du côté de l'imageur correspondant à la deuxième direction, N est le plus petit entier pour lequel le recul est supérieur à 2/N fois la distance entre le générateur de rayon X et l'imageur.

Le dispositif est ensuite mis en place vis-à-vis de l'objet à inspecter (E3). En particulier, l'alignement du générateur de rayons X est réalisé vis-à-vis de l'imageur, et on positionne le générateur de rayons X à la distance adéquate de l'imageur.

Le nombre de déplacements selon la première et la deuxième direction pour couvrir le champ de vue est déterminé. Suit ensuite une étape d'obtention d'un ensemble matriciel de sous-images (E4).

Les sous-images sont transférées sur un système informatique tel qu'un ordinateur portable pour y être traitées. Divers paramètres sont renseignés dans le logiciel de traitement, tels que la distance D1 entre le générateur de rayons X et l'imageur, et la distance D3 entre le générateur de rayons X et le point le plus proche de l'objet.

Un élément d'intérêt est déterminé dans ledit ensemble de sous-images (E5).

Les sous-images sont traitées, par agrandissement homothétique et superposition partielle des sous-images représentant tout ou partie de l'élément d'intérêt, comme décrits en référence aux figures 9a à 9c. Cela permet la détermination d'un facteur d'étirement permettant la coïncidence des sous-images au niveau du point d'intérêt. (E6).

La profondeur de l'élément d'intérêt est ensuite déterminée en fonction du facteur d'étirement (E7). Les dimensions de l'élément d'intérêt peuvent également être déterminées, comme expliqué en référence à la figure 9c.

A titre d'exemple de réalisation, un dispositif conforme à l'invention a été réalisé en utilisant un imageur présentant une surface active rectangulaire de 40 cm par 28,5 cm. Ce dispositif est dimensionné de sorte à permettre la couverture d'un champ de vue de 120 cm par 114 cm. Le dispositif réalisé présente un mécanisme d'extension de son axe vertical permettant de radiographier en deux passes successives un champ de vue de 200 cm par 114 cm. Le dispositif est repliable et transportable sur une palette de 80 cm par 120 cm.

A titre de second exemple, un dispositif conforme à l'invention a été réalisé en utilisant un imageur présentant une surface active rectangulaire de 43 cm par 35 cm. Ce dispositif permet de radiographier un champ de vue de 175 cm par 172 cm. Ce dispositif est pliant et permet pour son transport d'aligner dans un même plan les éléments constitutifs des supports mis en oeuvre.

De nombreuses configurations permettant la mise en oeuvre de l'invention sont ainsi envisageables.

L'invention ainsi développée permet l'inspection radiographique tridimensionnelle d'objets, notamment d'objets qu'il n'est pas possible ou pas souhaitable de déplacer.

L'invention est par exemple applicable à des applications de sécurité dans lesquelles les objets à radiographier ne peuvent être déplacés vers des installations fixes et pour les cas où l'objet est de grande dimension. Pour ces applications, la minimisation du temps d'intervention et de la dose de rayons X déposée sur l'objet sont des critères importants. Par ailleurs les sous-ensembles d'objet à analyser sont multi-centimétriques. Pour ces applications, il est optimum (mais pas absolument nécessaire) d'utiliser l'invention avec N=2.

L'inspection par radiographie de véhicules immobiles n'avait jusqu'alors pas de solution ergonomique. Sur des objets d'une telle longueur, il est possible de réaliser une radiographie sur l'axe transversal mais il est souvent impossible de la réaliser sur l'axe longitudinal tout en ayant une information de profondeur dans le véhicule. L'invention permet de remédier à ce problème en autorisant l'accès à la profondeur des sous-ensembles sur une seule radiographie sur l'axe transversal.

La radiographie mobile de conteneurs de dimensions standards (conteneur dits « ISO ») est envisageable dans des séquences automatisées et les objets pyrotechniques de grandes dimensions peuvent être inspectés de l'extérieur sans prendre le risque d'utiliser un endoscope en première intention.

L'invention permet des contrôles non destructifs, notamment pour les pièces imposantes comme des ailes d'avion. Pour de tels contrôles, la mise en oeuvre de l'invention avec N=3 est généralement une solution adéquate.

L'invention permet enfin de répondre à des problématiques techniques qui ne sont pas résolues de manière satisfaisante dans l'état de la technique, telles que la radiographie de grands animaux (chevaux, vaches, etc.) par un vétérinaire itinérant.

## Revendications

1. Dispositif d'inspection tridimensionnelle d'un objet (101, 201, 501, 601, 701, 801) par rayons X, comportant un générateur de rayons X (104, 204, 504, 604, 704 ,804) et un imageur (103, 203, 503, 603, 703, 803) numérique rectangulaire ou carré en regard dudit générateur de rayons X (104, 204, 504, 604, 704,804),
dans lequel le générateur de rayons X (104, 204, 504, 604, 704, 804) et l'imageur (103, 203, 503, 603, 703,803) sont destinés à être espacés l'un de l'autre d'une distance correspondant au grandissement de 1, le générateur de rayons X étant positionné sur un axe orthogonal à un plan d'extension de l'imageur (103, 203, 503, 603, 703,803), passant par le centre de l'imageur (103, 203, 503, 603, 703,803),
et dans lequel le générateur de rayons X (104, 204, 504, 604, 704, 804) est mobile dans un plan parallèle audit plan d'extension de l'imageur (103, 203, 503, 603, 703,803), et l'imageur (103, 203, 503, 603, 703, 803) est mobile dans son plan d'extension,
le dispositif comportant en outre un moyen de contrôle de la position et du déplacement dudit générateur de rayons X (104, 204, 504, 604, 704,804) et dudit imageur (103, 203, 503, 603, 703, 803) configuré de sorte à déplacer le générateur de rayons X (104, 204, 504, 604, 704, 804) par déplacements unitaires selon une première direction ou une deuxième direction respectivement parallèles aux côtés de l'imageur (103, 203, 503, 603, 703,803), et pour maintenir à chaque déplacement unitaire la position relative de l'imageur (103, 203, 503, 603, 703, 803) par rapport audit générateur de rayons X (104, 204, 504, 604, 704, 804),
ledit moyen de contrôle étant configuré de sorte que chaque déplacement unitaire du générateur de rayons X (104, 204, 504, 604, 704, 804) et de l'imageur (103, 203, 503, 603, 703, 803), dans la première ou dans la deuxième direction, corresponde à une fraction entière du côté correspondant de l'imageur (103, 203, 503, 603, 703, 803), à savoir 1/N fois la dimension du coté correspondant à la première direction et 1/N fois la dimension du côté correspondant à la deuxième direction, N étant un entier naturel supérieur à 1, de sorte à obtenir par prises de vue successives après chaque déplacement unitaire un ensemble matriciel de sous-images se chevauchant dans le plan d'extension de l'imageur,
le dispositif étant **caractérisé en ce que**, chaque sous-image a un centre affecté de coordonnées dans un plan de grandissement égal à N, lesdites coordonnées étant déterminées selon la position du centre de l'imageur (103, 203, 503, 603, 703, 803) lors de la prise de ladite sous-image, et **en ce que** le dispositif comporte en outre des moyens de traitement d'image adaptés à réaliser un agrandissement homothétique de chaque sous-image, laissant invariantes les coordonnées du centre de chaque sous-image, lesdits moyens de traitement d'image étant en outre configurés pour déterminer un facteur d'étirement permettant la coïncidence des sous-images représentant tout ou partie d'un élément d'intérêt (102, 202) prédéfinie de l'image.

2. Dispositif selon la revendication 1, comportant en outre des moyens de détermination d'une profondeur et des dimensions de l'élément d'intérêt (102, 202) en fonction du facteur d'étirement déterminé.

3. Dispositif selon la revendication 1 ou la revendication 2, comportant un premier support et un deuxième support, dans lequel :
- le premier support porte l'imageur (103, 203, 503, 603, 703, 803),
- le deuxième support porte le générateur de rayons X (104, 204, 504, 604, 704, 804).

4. Dispositif selon la revendication 3 dans lequel le premier support comporte des roulettes motorisées, configurées pour déplacer l'imageur (103, 203, 503, 603, 703, 803) dans la première direction, et un moyen de positionnement dudit imageur (103, 203, 503, 603, 703, 803) dans la deuxième direction comportant un élément télescopique ou un bras le long duquel l'imageur (103, 203, 503, 603, 703,803) peut se translater, et le deuxième support comporte des roulettes motorisées, configurées pour déplacer le générateur de rayons X (104, 204, 504, 604, 704,804) dans la première direction, et un moyen de positionnement dudit générateur de rayons X (104, 204, 504, 604, 704, 804) dans la deuxième direction comportant un élément télescopique ou un bras le long duquel le générateur de rayons X (104, 204, 504, 604, 704,804) peut se translater.

5. Dispositif selon la revendication 3, dans lequel le premier support comporte un premier portique et le deuxième support comporte un second portique,
le premier portique comportant une première poutre s'étendant dans la première direction, et un élément portant l'imageur (103, 203, 503, 603, 703, 803) s'étendant dans la deuxième direction adapté à positionner ledit imageur (103, 203, 503, 603, 703, 803) dans ladite deuxième direction, ledit élément portant l'imageur (103, 203, 503, 603, 703,803) étant lié à la première poutre de sorte à pouvoir être translaté selon la première direction, ledit élément portant l'imageur (103, 203, 503, 603, 703, 803) étant un élément télescopique ou une seconde poutre le long de laquelle l'imageur (103, 203, 503, 603, 703, 803) peut se translater,
le deuxième portique ayant une configuration identique à celle du premier portique et portant le générateur de rayons X (104, 204, 504, 604, 704, 804) en lieu et place de l'imageur (103, 203, 503, 603, 703, 803) du premier portique.

6. Dispositif selon la revendication 3, dans lequel le premier support comporte un premier portique et le deuxième support comporte un second portique,
le premier portique comportant un premier élément télescopique s'étendant dans la première direction, et un second élément télescopique portant l'imageur (103, 203, 503, 603, 703, 803) s'étendant dans la deuxième direction adapté à positionner ledit imageur (103, 203, 503, 603, 703, 803) dans ladite deuxième direction, ledit deuxième élément télescopique étant lié au premier élément télescopique de sorte à être déplacé dans la première direction par extension ou rétractation dudit premier élément télescopique,
le deuxième portique ayant une configuration identique à celle du premier portique et portant le générateur de rayons X (104, 204, 504, 604, 704, 804) en lieu et place de l'imageur (103, 203, 503, 603, 703, 803) du premier portique.

7. Dispositif selon la revendication 5 ou la revendication 6, dans lequel le premier et le deuxième support comportent chacun un pied anti-basculement (702, 802) comportant une troisième poutre, liée rigidement sensiblement orthogonalement à la première poutre.

8. Dispositif selon l'une des revendications 3 à 7, comportant un dispositif d'alignement à laser (707) permettant l'alignement relatif des premier et deuxième supports.

9. Dispositif selon la revendication 1 ou la revendication 2, comportant une structure liant rigidement le générateur de rayons X (104, 204, 504, 604, 704, 804) et l'imageur (103, 203, 503, 603, 703, 803), ladite structure entre le générateur de rayons X (104, 204, 504, 604, 704, 804) et l'imageur (103, 203, 503, 603, 703, 803) étant portée par un support comportant un système de déplacement de ladite structure selon la première direction et selon la deuxième direction.

10. Dispositif selon la revendication 9, dans lequel le système de déplacement de la structure comporte des roulettes motorisées configurées pour déplacer la structure (502) dans la première direction, et un mécanisme comportant un élément télescopique lié à la structure ou un bras orienté selon ladite deuxième direction le long duquel la structure (502) peut se translater.

11. Procédé d'inspection tridimensionnelle d'un objet (101, 201, 501, 601, 701, 801) par rayons X, comportant les étapes de :
- fourniture d'un dispositif selon l'une des revendications 1 à 10 ;
- mise en place du dispositif vis-à-vis de l'objet (E3), le générateur de rayons X (104, 204, 504, 604, 704, 804) et l'imageur (103, 203, 503, 603, 703,803) étant espacés l'un de l'autre d'une distance correspondant au grandissement de 1 ;
- obtention d'un ensemble matriciel de sous-images (E4) ;
- détermination d'un élément d'intérêt (E5) de l'objet représenté dans ledit ensemble de sous- images ;
- détermination, par agrandissement homothétique et superposition partielle des sous-images représentant tout ou partie de l'élément d'intérêt (102, 202), d'un facteur d'étirement (E6) permettant la coïncidence des sous-images au niveau de l'élément d'intérêt ; et
- détermination d'une profondeur et des dimensions de l'élément d'intérêt (102, 202) en fonction du facteur d'étirement déterminé (E7).

12. Procédé selon la revendication 11, comportant en outre, entre la fourniture du dispositif et sa mise en place,
- une étape de détermination du champ de vue nécessaire pour radiographier l'objet (E1),
- une étape de détermination du recul disponible vis-à-vis d'une face avant de l'objet pour la mise en place du générateur de rayon X ; et
- une étape de détermination de N (E2) en fonction du recul disponible et des dimensions d'une zone d'intérêt contenant l'élément d'intérêt.

13. Procédé selon la revendication 12, dans lequel N est déterminé de sorte que:
- si la zone d'intérêt est de dimension supérieure dans la première direction à la fraction 1/N du côté de l'imageur (103, 203, 503, 603, 703, 803) correspondant à la première direction et est de dimension supérieure dans la deuxième direction à la fraction 1/N du côté de l'imageur (103, 203, 503, 603, 703, 803) correspondant à la deuxième direction, N est le plus petit entier, supérieur à 1, pour lequel le recul est supérieur à 1/N fois la distance entre le générateur de rayon X et l'imageur (103, 203, 503, 603, 703, 803),
- si la zone d'intérêt est de dimension inférieure dans la première direction à la fraction 1/N du côté de l'imageur (103, 203, 503, 603, 703, 803) correspondant à la première direction ou est de dimension inférieure dans la deuxième direction à la fraction 1/N du côté de l'imageur (103, 203, 503, 603, 703, 803) correspondant à la deuxième direction, N est le plus petit entier pour lequel le recul est supérieur à 2/N fois la distance entre le générateur de rayon X et l'imageur (103, 203, 503, 603, 703, 803).

14. Procédé selon la revendication 12 ou la revendication 13, dans lequel N=2 ou N=3, comprenant une étape de détermination du nombre de vues n nécessaires avec l'imageur (103, 203, 503, 603, 703,803) pour couvrir le champ de vue, sans superposition d'images, dans la première direction et du nombre de vues m nécessaires avec l'imageur (103, 203, 503, 603, 703,803) pour couvrir le champ de vue, sans superposition d'image, dans la deuxième direction, et dans lequel l'étape d'obtention de l'ensemble matriciel de sous-images comprend l'obtention de N.n-3 à N.n+1 sous-images dans la première direction et de N.m-3 à N.m+1 sous-images dans la deuxième direction.

## Patentansprüche

1. Vorrichtung zur dreidimensionalen Inspektion eines Objekts (101, 201, 501, 601, 701, 801) durch Röntgenstrahlen, enthaltend einen Röntgengenerator (104, 204, 504, 604, 704, 804) und einen rechteckigen oder quadratischen, digitalen Bildgeber (103, 203, 503, 603, 703, 803), der dem Röntgengenerator (104, 204, 504, 604, 704, 804) gegenüberliegt,
wobei der Röntgengenerator (104, 204, 504, 604, 704, 804) und der Bildgeber (103, 203, 503, 603, 703, 803) in einem Abstand voneinander angeordnet sein sollen, der der Vergrößerung 1 entspricht, wobei der Röntgengenerator auf einer Achse orthogonal zu einer durch das Zentrum des Bildgebers (103, 203, 503, 603, 703, 803) verlaufenden Erstreckungsebene des Bildgebers (103, 203, 503, 603, 703, 803) positioniert ist,
und wobei der Röntgengenerator (104, 204, 504, 604, 704, 804) in einer Ebene parallel zu der Erstreckungsebene des Bildgebers (103, 203, 503, 603, 703, 803) beweglich ist und der Bildgeber (103, 203, 503, 603, 703, 803) in seiner Erstreckungsebene beweglich ist,
wobei die Vorrichtung ferner ein Mittel zum Steuern der Position und Verlagerung des Röntgengenerators (104, 204, 504, 604, 704, 804) und des Bildgebers (103, 203, 503, 603, 703, 803) enthält, das dazu ausgelegt ist, den Röntgengenerator (104, 204, 504, 604, 704, 804) durch einheitliche Verlagerungen in einer ersten Richtung oder einer zweiten Richtung jeweils parallel zu den Seiten des Bildgebers (103, 203, 503, 603, 703, 803) zu verlagern und bei jeder einheitlichen Verlagerung die relative Position des Bildgebers (103, 203, 503, 603, 703, 803) in Bezug auf den Röntgengenerator (104, 204, 504, 604, 704, 804) beizubehalten,
wobei das Steuermittel dazu ausgelegt ist, dass jede einheitliche Verlagerung des Röntgengenerators (104, 204, 504, 604, 704, 804) und des Bildgebers (103, 203, 503, 603, 703, 803) in der ersten oder zweiten Richtung einem ganzzahligen Bruchteil der entsprechenden Seite des Bildgebers (103, 203, 503, 603, 703, 803) entspricht, nämlich dem 1/N-fachen der Abmessung der Seite, die der ersten Richtung entspricht, und dem 1/N-fachen der Abmessung der Seite, die der zweiten Richtung entspricht, wobei N eine natürliche ganze Zahl größer als 1 ist, um durch aufeinanderfolgende Bildaufnahmen nach jeder einheitlichen Verlagerung eine Matrixanordnung von überlappenden Unterbildern in der Erstreckungsebene des Bildgebers zu erhalten,
wobei die Vorrichtung **dadurch gekennzeichnet ist, dass**
jedes Unterbild ein Zentrum hat, dem Koordinaten in einer Vergrößerungsebene gleich N zugeordnet sind, wobei die Koordinaten in Abhängigkeit von der Position des Zentrums des Bildgebers (103, 203, 503, 603, 703, 803) beim Aufnehmen des Unterbildes bestimmt werden, und dass
die Vorrichtung ferner Bildverarbeitungsmittel enthält, die dazu ausgelegt sind, eine homothetische Vergrößerung jedes Unterbildes zu erhalten, wobei die Koordinaten des Zentrums jedes Unterbildes unveränderlich bleiben, wobei die Bildverarbeitungsmittel ferner dazu ausgelegt ist, einen Streckungsfaktor zu ermitteln, der die Deckungsgleichheit von Unterbildern ermöglicht, die ein vorgegebenes, interessierendes Element (102, 202) des Bildes ganz oder teilweise darstellen.

2. Vorrichtung nach Anspruch 1, ferner enthaltend Mittel zum Bestimmen von einer Tiefe und von Abmessungen des interessierenden Elements (102, 202) in Abhängigkeit von dem ermittelten Streckungsfaktor.

3. Vorrichtung nach Anspruch 1 oder Anspruch 2, enthaltend einen ersten Träger und einen zweiten Träger, wobei:
- der erste Träger den Bildgeber (103, 203, 503, 603, 703, 803) trägt,
- der zweite Träger den Röntgengenerator (104, 204, 504, 604, 704, 804) trägt.

4. Vorrichtung nach Anspruch 3, wobei der erste Träger motorisch angetriebene Laufrollen enthält, die dazu ausgelegt sind, den Bildgeber (103, 203, 503, 603, 703, 803) in der ersten Richtung zu verlagern, sowie ein Mittel zum Positionieren des Bildgebers (103, 203, 503, 603, 703, 803) in der zweiten Richtung, das ein Teleskopteil oder einen Arm enthält, entlang dessen der Bildgeber (103, 203, 503, 603, 703, 803) sich verschieben kann, und wobei der zweite Träger motorisch angetriebene Laufrollen enthält, die dazu ausgelegt sind, den Röntgengenerator (104, 204, 504, 604, 704, 804) in der ersten Richtung zu verlagern, sowie ein Mittel zum Positionieren des Röntgengenerators (104, 204, 504, 604, 704, 804) in der zweiten Richtung, das ein Teleskopteil oder einen Arm enthält, entlang dessen der Röntgengenerator (104, 204, 504, 604, 704, 804) sich verschieben kann.

5. Vorrichtung nach Anspruch 3, wobei der erste Träger ein erstes Portal enthält und der zweite Träger ein zweites Portal enthält,
wobei das erste Portal einen ersten Balken enthält, der sich in der ersten Richtung erstreckt, sowie ein den Bildgeber (103, 203, 503, 603, 703, 803) tragendes Element, das sich in der zweiten Richtung erstreckt und dazu geeignet ist, den Bildgeber (103, 203, 503, 603, 703, 803) in der zweiten Richtung zu positionieren, wobei das den Bildgeber (103, 203, 503, 603, 703, 803) tragende Element mit dem ersten Balken so verbunden ist, dass es in der ersten Richtung verschiebbar ist, wobei das den Bildgeber (103, 203, 503, 603, 703, 803) tragende Element ein Teleskopteil oder ein zweiter Balken ist, entlang dessen der Bildgeber (103, 203, 503, 603, 703, 803) sich verschieben kann,
wobei das zweite Portal eine Gestalt hat, die mit der des ersten Portals identisch ist, und den Röntgengenerator (104, 204, 504, 604, 704, 804) anstelle des Bildgebers (103, 203, 503, 603, 703, 803) des ersten Portals trägt.

6. Vorrichtung nach Anspruch 3, wobei der erste Träger ein erstes Portal enthält und der zweite Träger ein zweites Portal enthält,
wobei das erste Portal ein erstes Teleskopteil enthält, das sich in der ersten Richtung erstreckt, sowie ein den Bildgeber (103, 203, 503, 603, 703, 803) tragendes zweites Teleskopteil, das sich in der zweiten Richtung erstreckt und dazu geeignet ist, den Bildgeber (103, 203, 503, 603, 703, 803) in der zweiten Richtung zu positionieren, wobei das zweite Teleskopteil mit dem ersten Teleskopteil so verbunden ist, dass es durch Aus- oder Einfahren des ersten Teleskopteils in der ersten Richtung verlagert wird,
wobei das zweite Portal eine Gestalt hat, die mit der des ersten Portals identisch ist, und den Röntgengenerator (104, 204, 504, 604, 704, 804) anstelle des Bildgebers (103, 203, 503, 603, 703, 803) des ersten Portals trägt.

7. Vorrichtung nach Anspruch 5 oder Anspruch 6, wobei der erste und der zweite Träger jeweils einen Antikippfuß (702, 802) enthalten, der einen dritten Balken aufweist, der im Wesentlichen orthogonal mit dem ersten Balken starr verbunden ist.

8. Vorrichtung nach einem der Ansprüche 3 bis 7, enthaltend eine Laserausrichtvorrichtung (707), welche die relative Ausrichtung des ersten und des zweiten Trägers ermöglicht.

9. Vorrichtung nach Anspruch 1 oder Anspruch 2, enthaltend eine Struktur, die den Röntgengenerator (104, 204, 504, 604, 704, 804) und den Bildgeber (103, 203, 503, 603, 703, 803) starr verbindet, wobei die Struktur zwischen dem Röntgengenerator (104, 204, 504, 604, 704, 804) und dem Bildgeber (103, 203, 503, 603, 703, 803) liegt und von einem Träger getragen ist, der ein System zum Verlagern der Struktur in der ersten Richtung und in der zweiten Richtung enthält.

10. Vorrichtung nach Anspruch 9, wobei das System zum Verlagern der Struktur motorisch angetriebene Laufrollen enthält, die dazu ausgelegt sind, die Struktur (502) in der ersten Richtung zu verlagern, sowie einen Mechanismus, der ein mit der Struktur verbundenes Teleskopteil oder einen Arm enthält, der in der zweiten Richtung ausgerichtet ist und entlang dessen die Struktur (502) sich verschieben kann.

11. Verfahren zur dreidimensionalen Inspektion eines Objekts (101, 201, 501, 601, 701, 801) durch Röntgenstrahlen, umfassend die Schritte:
- Bereitstellen einer Vorrichtung nach einem der Ansprüche 1 bis 10;
- Platzieren der Vorrichtung gegenüber dem Objekt (E3), wobei der Röntgengenerator (104, 204, 504, 604, 704, 804) und der Bildgeber (103, 203, 503, 603, 703, 803) in einem Abstand voneinander angeordnet sind, der der Vergrößerung 1 entspricht;
- Erhalten einer Matrixanordnung von Unterbildern (E4);
- Bestimmen eines interessierenden Elements (E5) des Objekts, das in der Anordnung von Unterbildern dargestellt ist;
- Ermitteln eines Streckungsfaktors (E6), der die Deckungsgleichheit der Unterbilder im Bereich des interessierenden Elements ermöglicht, durch homothetische Vergrößerung und teilweise Überlagerung der Unterbilder, die das interessierende Element (102, 202) ganz oder teilweise darstellen; und
- Bestimmen von einer Tiefe und von Abmessungen des interessierenden Elements (102, 202) in Abhängigkeit von dem ermittelten Streckungsfaktor (E7).

12. Verfahren nach Anspruch 11, ferner umfassend zwischen dem Bereitstellen der Vorrichtung und deren Platzieren,
- einen Schritt des Bestimmens des für die Röntgenaufnahme des Objekts erforderlichen Sichtfelds (E1),
- einen Schritt des Bestimmens der verfügbaren Entfernung gegenüber einer Vorderseite des Objekts für die Platzierung des Röntgengenerators; und
- einen Schritt des Bestimmens von N (E2) in Abhängigkeit von der verfügbaren Entfernung und von den Abmessungen eines interessierenden Bereichs, der das interessierende Element enthält.

13. Verfahren nach Anspruch 12, wobei N so bestimmt wird, dass
- dann, wenn der interessierende Bereich in der ersten Richtung größer bemessen ist als der Bruchteil 1/N der der ersten Richtung entsprechenden Seite des Bildgebers (103, 203, 503, 603, 703, 803) und in der zweiten Richtung größer ist als der Bruchteil 1/N der der zweiten Richtung entsprechenden Seite des Bildgebers (103, 203, 503, 603, 703, 803), N die kleinste ganze Zahl größer als 1 ist, für die die Entfernung größer als das 1/N-fache des Abstands zwischen dem Röntgengenerator und dem Bildgeber (103, 203, 503, 603, 703, 803) ist,
- dann, wenn der interessierende Bereich in der ersten Richtung kleiner bemessen ist als der Bruchteil 1/N der der ersten Richtung entsprechenden Seite des Bildgebers (103, 203, 503, 603, 703, 803) oder in der zweiten Richtung kleiner bemessen ist als der Bruchteil 1/N der der zweiten Richtung entsprechenden Seite des Bildgebers (103, 203, 503, 603, 703, 803), N die kleinste ganze Zahl ist, für die die Entfernung größer als das 2/N-fache des Abstands zwischen dem Röntgengenerator und dem Bildgeber (103, 203, 503, 603, 703, 803) ist.

14. Verfahren nach Anspruch 12 oder Anspruch 13, wobei N = 2 oder N = 3, umfassend einen Schritt des Bestimmens der Anzahl von Ansichten n, die mit dem Bildgeber (103, 203, 503, 603, 703, 803) benötigt werden, um das Sichtfeld ohne Bildüberlagerung in der ersten Richtung abzudecken, und der Anzahl von Ansichten m, die mit dem Bildgeber (103, 203, 503, 603, 703, 803) benötigt werden, um das Sichtfeld ohne Bildüberlagerung in der zweiten Richtung abzudecken, und wobei der Schritt des Erhaltens der Matrixanordnung von Unterbildern das Erhalten von N.n-3 bis N.n+1 Unterbildern in der ersten Richtung und von N.m-3 bis N.m+1 Unterbildern in der zweiten Richtung umfasst.

## Claims

1. An apparatus for three-dimensional inspection (101, 201, 501, 601, 701, 801) of an object by X-rays, comprising an X-ray generator (104, 204, 504, 604, 704, 804) and a rectangular or square digital imaging device (103, 203, 503, 603, 703, 803) facing said X-ray generator (104, 204, 504, 604, 704,804).
in which the X-ray generator (104, 204, 504, 604, 704, 804) and the imaging device (103, 203, 503, 603, 703,803) are configured to be spaced from each other by a distance corresponding to a magnification by 1, the X-ray generator being positioned on an axis orthogonal to a plane of extension of the imaging device (103, 203, 503, 603, 703,803), passing through the center of the imaging device (103, 203, 503, 603, 703,803),
and in which the X-ray generator (104, 204, 504, 604, 704, 804) is movable in a plane parallel to said plane of extension of the imaging device (103, 203, 503, 603, 703,803), and the imaging device (103, 203, 503, 603, 703, 803) is movable within its plane of extension,
the apparatus further comprising a means for controlling the position and the movement of said X-ray generator (104, 204, 504, 604, 704, 804) and of said imaging device (103, 203, 503, 603, 703, 803) configured so as to move the X-ray generator (104, 204, 504, 604, 704, 804) by unitary movements in a first direction or a second direction respectively parallel to the sides of the imaging device (103, 203, 503, 603, 703,803), and, at each unitary movement, to hold the relative position of the imaging device (103, 203, 503, 603, 703, 803) in relation to said X-ray generator (104, 204, 504, 604, 704, 804),
said control means being configured such that each unitary movement of the X-ray generator (104, 204, 504, 604, 704, 804) and of the imaging device (103, 203, 503, 603, 703, 803), in the first or in the second direction, corresponds to an integer number fraction of the corresponding side of the imaging device (103, 203, 503, 603, 703, 803), that is to say 1/N times the dimension of the side corresponding to the first direction and 1/N times the dimension of the side corresponding to the second direction, N being a natural number greater than 1, so as to obtain, by taking successive shots after each unitary movement, a matrix set of sub-images overlapping in the plane of extension of the imaging device,
the apparatus being **characterized in that**, each sub-image has a center attributed with coordinates in a plane of magnification equal to N, said coordinates being determined according to the position of the center of the imaging device (103, 203, 503, 603, 703, 803) on taking said sub-image, and **in that** the apparatus further comprises image processing means configured to perform a homothetic magnification of each sub-image, leaving unchanged the coordinates of the center of each sub-image, said image processing means being furthermore configured to determine a stretch factor enabling the coincidence of the sub-images representing all or some of a predefined element of interest (102, 202) of the image.

2. An apparatus according to claim 1, further comprising means for determining a depth and dimensions of the element of interest (102, 202) according to the determined stretch factor.

3. An apparatus according to claim 1 or claim 2, comprising a first mounting and a second mounting, in which:
- the first mounting bears the imaging device (103, 203, 503, 603, 703, 803),
- the second mounting bears the X-ray generator (104, 204, 504, 604, 704, 804).

4. An apparatus according to claim 3, in which the first mounting comprises motor-driven wheels, configured to move the imaging device (103, 203, 503, 603, 703, 803) in the first direction, and a positioning means for positioning said imaging device (103, 203, 503, 603, 703, 803) in the second direction comprising a telescopic member or an arm along which the imaging device (103, 203, 503, 603, 703,803) can translate, and the second mounting comprises motor-driven wheels, configured to move the X-ray generator (104, 204, 504, 604, 704,804) in the first direction, and a positioning means for positioning said X-ray generator (104, 204, 504, 604, 704, 804) in the second direction comprising a telescopic member or an arm along which the X-ray generator (104, 204, 504, 604, 704, 804) can translate.

5. An apparatus according to claim 3, in which the first mounting comprises a first gantry and the second mounting comprises a second gantry,
the first gantry comprising a first beam extending in the first direction and a member bearing the imaging device (103, 203, 503, 603, 703, 803) extending in the second direction configured to position said imaging device (103, 203, 503, 603, 703, 803) in said second direction, said member bearing the imaging device (103, 203, 503, 603, 703,803) being linked to the first beam so as to be able to be translated in the first direction, said member bearing the imaging device (103, 203, 503, 603, 703, 803) being a telescopic member or a second beam along which the imaging device (103, 203, 503, 603, 703, 803) is able to translate,
the second gantry having a configuration identical to that of the first gantry and bearing the X-ray generator (104, 204, 504, 604, 704, 804) instead of the imaging device (103, 203, 503, 603, 703, 803) of the first gantry.

6. An apparatus according to claim 3, in which the first mounting comprises a first gantry and the second mounting comprises a second gantry,
the first gantry comprising a first telescopic member extending in the first direction and a second telescopic member bearing the imaging device (103, 203, 503, 603, 703, 803) extending in the second direction configured to position said imaging device (103, 203, 503, 603, 703, 803) in said second direction, said second telescopic member being linked to the first telescopic member so as to be moved in the first direction by elongation or retraction of said first telescopic member,
the second gantry having a configuration identical to that of the first gantry and bearing the X-ray generator (104, 204, 504, 604, 704, 804) instead of the imaging device (103, 203, 503, 603, 703, 803) of the first gantry.

7. An apparatus according to claim 5 or claim 6 in which the first and the second mounting each comprise an anti-tipping foot (702, 802) comprising a third beam, rigidly linked substantially orthogonally to the first beam.

8. An apparatus according to one of claims 3 to 7, comprising a laser aligning device (707) enabling the relative alignment of the first and second mountings.

9. An apparatus according to claim 1 or claim 2, comprising a structure rigidly linking the X-ray generator (104, 204, 504, 604, 704, 804) and the imaging device (103, 203, 503, 603, 703, 803), said structure between the X-ray generator (104, 204, 504, 604, 704, 804) and the imaging device (103, 203, 503, 603, 703, 803) being carried by a mounting comprising a system for moving said structure in the first direction and in the second direction.

10. An apparatus according to claim 9, in which the system for moving the structure comprises motor-driven wheels configured to move the structure (502) in the first direction, and a mechanism comprising a telescopic member linked to the structure or an arm oriented in said second direction along which the structure (502) is able to translate.

11. A method of three-dimensional inspection of an object (101, 201, 501, 601, 701, 801) by X-rays comprising the steps of:
- providing an apparatus according to one of claims 1 to 10;
- putting the apparatus in place in relation to the object (S3), the X-ray generator (104, 204, 504, 604, 704, 804) and the imaging device (103, 203, 503, 603, 703,803) being spaced from each other by a distance corresponding to a magnification by 1;
- obtaining a matrix set of sub-images (S4);
- determining an element of interest (S5) of the object represented in said set of sub-images;
- determining, by homothetic magnification and partial superposition of the sub-images representing all or part of the element of interest (102, 202), a stretch factor (S6) enabling the coincidence of the sub-images at the location of the element of interest; and
- determining a depth and dimensions of the element of interest (102, 202) according to the determined stretch factor (S7).

12. A method according to claim 11, further comprising, between providing the apparatus and putting it in place,
- a step of determining the field of view necessary to X-ray the object (S1),
- a step of determining the available spacing away in relation to a front face of the object for the putting in place of the X-ray generator; and
- a step of determining N (S2) according to the available spacing away and the dimensions of a region of interest containing the element of interest.

13. A method according to claim 12, in which N is determined such that:
- if the region of interest is of greater dimension in the first direction than the fraction 1/N of the side of the imaging device (103, 203, 503, 603, 703, 803) corresponding to the first direction and is of greater dimension in the second direction than the fraction 1/N of the side of the imaging device (103, 203, 503, 603, 703, 803) corresponding to the second direction, N is the smallest integer, greater than 1, for which the spacing away is greater than 1/N times the distance between the X-ray generator and the imaging device (103, 203, 503, 603, 703, 803),
- if the region of interest is of smaller dimension in the first direction than the fraction 1/N of the side of the imaging device (103, 203, 503, 603, 703, 803) corresponding to the first direction or is of smaller dimension in the second direction than the fraction 1/N of the side of the imaging device (103, 203, 503, 603, 703, 803) corresponding to the second direction, N is the smallest integer for which the spacing away is greater than 2/N times the distance between the X-ray generator and the imaging device (103, 203, 503, 603, 703, 803).

14. A method according to claim 12 or claim 13, in which N=2 or N=3, comprising a step of determining the number of shots n necessary with the imaging device (103, 203, 503, 603, 703, 803) to cover the field of view, without superposition of images, in the first direction and the number of shots m necessary with the imaging device (103, 203, 503, 603, 703, 803) to cover the field of view, without superposition of images, in the second direction, and in which the step of obtaining the matrix set of sub-images comprises obtaining N.n-3 to N.n+1 sub-images in the first direction and N.m-3 to N.m+1 sub-images in the second direction.
